(19) Europäisches Patentamt
European Patent Office
Office européen des brevets

(11) Publication number: 0 302 694 A2

# (12) EUROPEAN PATENT APPLICATION

(21) Application number: 88307115.1

(22) Date of filing: 02.08.88

(51) Int. Cl.4: C 07 C 125/065

(30) Priority: 05.08.87 CS 5824/87

(43) Date of publication of application:
08.02.89 Bulletin 89/06

(84) Designated Contracting States:
BE CH DE GB LI NL SE

(71) Applicant: SLOVENSKA AKADEMIA VIED
Obráncov mieru 49
Bratislava (CS)

(72) Inventor: Csollei, Josef
No 10 Rokosovskeho
Bratislava (CS)

Borovansky, Alois
No 34 Cihlarska
Brno (CS)

Benes, Ludek
No 39 Brnianska
Bratislava (CS)

Berezekova, Marianna
No 30 Budoneho
Bratislava (CS)

Bederova, Eva
No 14 Nabrezna
Bratislava (CS)

Svec, Pavek
No 8 Korenicova
Bratislava (CS)

Bauer, Viktor
No 17 Bratislavska
Samorin (CS)

(74) Representative: Fitzpatrick, Alan James et al
Fitzpatricks 4 West Regent Street
Glasgow G2 1RS Scotland (GB)

## (54) Substituted aryloxyamino-propanols and methods of preparing same.

(57) Betaadrenolytically effective substances consisting of 1-aryloxy-3-alkylamino-2-propanols of the general formula I

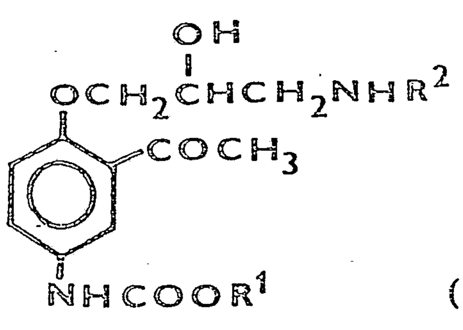

(I)

wherein $R^1$ is alkyl with 3 to 4 carbon atoms, and $R^2$ is a ramified alkyl with 3 to 4 carbon atoms, such substances being prepared in a reaction in a polar solvent from

(a) alkyl ester of 3-acetyl-4[(3-bromo-2-hydroxy)-propoxy]phenylcarbamic acid with a primary alkylamine;

(b) alkyl ester of 3-acetyl-4[(2-3,epoxy)-propoxy]phenylcarbamic acid with a primary alkylamine in the medium of an alkanol with 1 to 3 carbon atoms;

(c) alkyl ester of 3-acetyl-4-hydroxyphenylcarbamic acid with 1-alkylamino-2,3-epoxypropane, the reaction being catalyzed by an alkali metal hydroxide; and

(d) alkyl ester of 3-acetyl-4-hydroxyphenylcarbamic acid with 1-alkylamino-3-chloro-2-propanol, the reaction being catalyzed by an alkali metal hydroxide.

EP 0 302 694 A2

## Description

### SUBSTITUTED ARYLOXYAMINOPROPANOLS AND METHODS OF PREPARING SAME

The invention relates to 1-aryloxy-3-amino-2-propanols of the general formula I

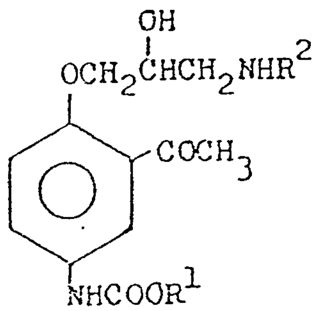

(I)

wherein $R^1$ is alkyl with 3 to 4 carbon atoms, and $R^2$ is a ramified alkyl with 3 to 4 carbon atoms, as well as to a method of preparing said substances.

Substituted aryloxyaminopropanols of the general formula I, as defined above are new undescribed compounds, not found in the relevant literature hitherto, which exhibit typical effects of betaadrenolytics, similarly as with aryloxyaminopropanols substituted on the benzene ring in the ortho-, meta- and para- position by an alkoxy-carbonylamino substituent (Czechoslovak Authorship Certificate No. 220,271).

The prepared substances were tested on betaadrenolytic and antiarrhythmic properties and compared with standard ones, viz. practolol and pindolol. It has been found from the negatively chronotropic effect evaluation, that all of the substances reduce the pulse frequency, on average, by 7 to 17 per cent. The force of contraction of spontaneously pulsating auricles was significantly raised by pindolol, practolol and the substance of the general formula I in which $R^1$ stands for propyl, and $R^2$ for isopropyl group. A cumulative curve (both chronotropic and inotropic) expressing the dependence of the effect upon the isoprenaline dose was being markedly shifted to the right also by applying the substance of the general formula I with the above meanings of $R^1$ and $R^2$ and with the $pA_2$ values of 6.8 and 8.2.

Substituted aryloxyaminopropanols of the general formula I as defined hereinbefore can be prepared, according to the invention, in different ways, such as, for instance, that the following substances, respectively, are allowed to react in a polar solvent:

(a) alkyl ester of 3-acetyl-4-[(3-bromo-2-hydroxy)-propoxy]phenylcarbamic acid of the general formula II

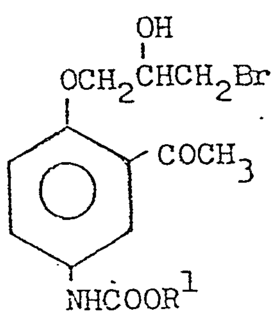

(II)

wherein $R^1$ has the same meaning as in the formula I, with a primary alkylamine of the general formula $NH_2R^2$ in which $R^2$ has the same meaning as in the formula I, preferably in aqueous medium, for one to five days at room temperature;

(b) alkyl ester of 3-acetyl-4[(2,3-epoxy)-propoxy]phenylcarbamic acid of the general formula III

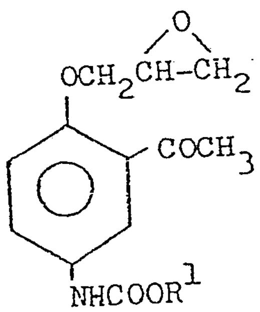

(III)

wherein $R^1$ has the same meaning as in the formula I, with a primary alkylamine of the general formula $NH_2R^2$ in which $R^2$ has the same meaning as in the formula I, preferably in a medium of an alkanol with 1 to 6 carbon atoms, preferably ethanol, at the boiling temperature of the reaction mixture;

(c) alkyl ester of 3-acetyl-4-hydroxyphenylcarbamic acid of the general formula IV

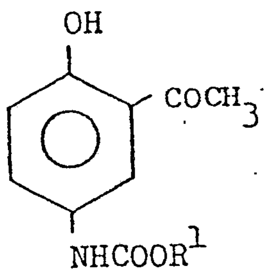

(IV)

in which $R^1$ has the same meaning as in the formula I, with 1-alkylamino-2,3-epoxypropane of the general formula V

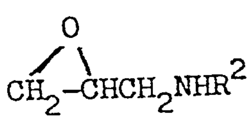

(V)

in which $R^2$ has the same meaning as in the formula I, preferably in aqueous medium, the reaction being catalyzed by an alkali metal hydroxide such as e.g. sodium or potassium hydroxide;

(d) alkyl ester of 3-acetyl-4-hydroxyphenylcarbamic acid of the general formula IV in which $R^1$ has the same meaning as in the formula I, with 1-alkylamino-3-chloro-2-propanol of the general formula VI

$ClCH_2\overset{OH}{CH}CH_2NHR^2$ (VI)

in which $R^2$ has the same meaning as in the general formula I, preferably in ethanolic medium, the reaction being catalyzed by an alkali metal hydroxide such as e.g. sodium hydroxide, preferably in a sealed tube or an autoclave at a temperature of from 100 to 120°C.

After the reaction according to one of the above variants (a), (b), (c) or (d), respectively, the reaction mixture is mostly treated in such a way that the raw product, after the organic solvent has been distilled off, is dissolved in diluted hydrochloric acid, shaken with ether, and the base released by alkalinisation from the separated aqueous phase is extracted into ether. From this solution, the basic ester can be immediately converted to the desired salt whereupon the latter can be cleaned by crystallizing from an organic solvent or a blend of such solvents.

Among organic acid salts there may be considered, for example, hydrochloride, hydrobromide, sulphate, phosphate, and among inorganic acid salts e.g. oxalate, maleinate, fumarate and so forth.

Details of the process are given in the following examples.

EXAMPLE 1

Propyl ester of 3-acetyl-4-[(2-hydroxy-3-isopropylamino)propoxy]phenylcarbamic acid

To 2.3 g (0.04 mol) of isopropylamine in 10 ml water there were successively added 6.5 g (0.02 mol) of propyl ester of 3-acetyl-4-[(3-bromo-2-hydroxy)-propoxy]phenylcarbamic acid (Czechoslovak Authorship Certificate No. (PV 1890-87)), the mixture having been agitated for 24 hours at room temperature. After the reaction, excessive isopropylamine was distilled off the reaction mixture in vacuo whereupon the evaporation residue was dissolved in diluted hydrochloric acid, three times shaken with ether, and from the separated aqueous phase there was released by alkalinization the base which was extracted into ether. After drying by anhydrous magnesium sulphate, the hydrochloride was separated from the filtrate by adding an etheric solution of dry hydrogen chloride, the hydrochloride being recrystallized out of the acetone/ethanol mixture (melting point 163 - 165°C).

EXAMPLE 2

Propyl ester of 3-acetyl-4[(2-hydroxy-3-tert.butylamino)propoxy]phenylcarbamic acid

To a solution of 10.2 g (0.03 mol) of propyl ester of 3-acetyl-4[(2,3-epoxy)-propoxy]phenylcarbamic acid (Czechoslovak Authorship Certificate No. (PV 1891-87)) in 30 ml of ethanol, there was added 3.5 g (0.06 mol) of tert. butylamine and 2 ml water, the mixture having been heated for 24 hours at the reaction mixture boiling temperature. The solvent and an amine excess were distilled off in vacuo. The procedure of Example 1 was then followed. The separated hydrochloride was re-crystallized from ethanol (melting point 181 - 183°C).

EXAMPLE 3

Butyl ester of 3-acetyl-4[(2-hydroxy-3-tert.butylamino)propoxy]phenylcarbamic acid

15.0 g (0.06 mol) of butyl ester of 3-acetyl-4-hydroxyphenylcarbamic acid (Czechoslovak Authorship Certificate No. (PV 9795-86)) and 7.7 g (0.06 mol) of 1-tert.butylamino-2,3-epoxypropane in 40 ml water together with sodium hydroxide as catalyst, were mixed for 6 hours at room temperature. A clear solution was decanted and a bituminous layer was dissolved in diluted hydrochloric acid, shaken three times with ether, and the base released by alkalinization from the separated aqueous phase was extracted into ether. After drying by anhydrous magnesium sulphate, and adding an etheric oxalic acid solution, the oxalate was separated and recrystallized from acetone (melting point 227 - 230°C).

EXAMPLE 4

Butyl ester of 3-acetyl-4[(2-hydroxy-3-isopropylamino)propoxy]phenylcarbamic acid

5.0 g (0.02 mol) of butyl ester of 3-acetyl-4-hydroxyphenylcarbamic acid (Czechoslovak Authorship Certificate No. (PV 9795-86)), 3.7 g (0.02 mol) of hydrochloride of 1-chloro-3-isopropylamino-2-propanol and 1.9 g (0.05 mol) of sodium hydroxide in 75 ml ethanol were heated in a sealed tube for 10 hours at 100°C. The solvent was distilled off the reaction mixture in vacuo, and a residue was suspended in 30 ml of 2N sodium hydroxide. The released base was shaken into ether and after drying by anhydrous magnesium sulphate and adding an etheric solution of dry hydrogen chloride the hydrochloride was separated from the filtrate and then recrystallized from 2-propanol (melting point 138 - 141°C).

By using the above described methods the following new, unpublished substances were prepared:

(1) Propyl ester of 3-acetyl-4-[(2-hydroxy-3-isopropylamino)-propoxy]phenylcarbamic acid:
as hydrochloride, melting point 163 - 165°C (acetoneethanol) according to EXAMPLES 1, 2, 3 and 4
Anal.: $C_{18}H_{29}N_2O_5Cl$
theory/actual value
C 55.59/55.53
H 7.51/ 7.57
N 7.20/ 7.01
IR (KBr) C=O (1675, 1695 $cm^{-1}$), C=C (1590, 1615 $cm^{-1}$), N-H (3310 $cm^{-1}$).

(2) Butyl ester of 3-acetyl-4-[(2-hydroxy-3-isopropylamino)propoxy]phenylcarbamic acid:
as hydrochloride, melting point 138 - 141°C (2-propanol) according to EXAMPLES 1, 2, 3 and 4
Anal.: $C_{19}H_{31}N_2O_5Cl$
theory/actual value
C 56.62/56.48
H 7.75/ 7.97

N 6.95/ 7.03
IR (KBr) C=O (1675, 1695 $cm^{-1}$), C=C (1590, 1612 $cm^{-1}$), N-H (3310 $cm^{-1}$).

(3) Propyl ester of 3-acetyl-4-[(2-hydroxy-3-tert.butylamino)-propoxy]phenylcarbamic acid:
as hydrochloride, melting point 181 - 183°C (ethanol) according to EXAMPLES 1, 2, 3 and 4
Anal.: $C_{19}H_{31}N_2O_5Cl$
theory/actual value
C 56.63/56.65
H 7.75/8.00
N 6.95/6.75
IR (KBr) C=O (1665, 1725 $cm^{-1}$), C=C (1592, 1615 $cm^{-1}$), N-H (3305 $cm^{-1}$).

(4) Butyl ester of 3-acetyl-4-[(2-hydroxy-3-tert.butylamino)-propoxy]phenylcarbamic acid:
as oxalate, m.p. 227 - 230°C
according to EXAMPLES 1, 2, 3 and 4
Anal.: $C_{22}H_{34}N_2O_9$
theory/actual value
C 56.16/55.92
H 7.28/ 6.93
N 5.95/ 5.56
IR (KBr) C=O (1670, 1750 $cm^{-1}$), C=C (1590, 1610 $cm^{-1}$), N-H (3315 $cm^{-1}$).

**Claims**

1. Substituted 1-aryloxy-3-amino-2-propanols of the general formula I

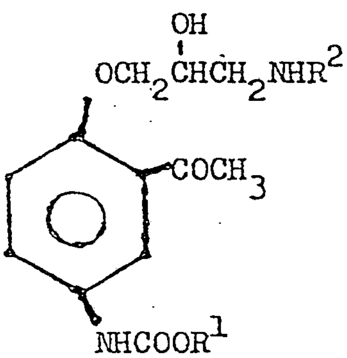

(I)

wherein $R^1$ is alkyl with 3 to 4 carbon atoms, and $R^2$ is a ramified alkyl with 3 to 4 carbon atoms, as well as salts thereof with both inorganic and organic acids.

2. A method of preparing substituted aryloxyaminopropanols of the general formula I according to claim 1, characterised in that an alkyl ester of 3-acetyl-4[(3-bromo-2-hydroxy)propoxy]phenylcarbamic acid of the general formula II

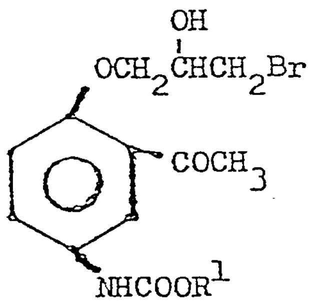

(II)

in which $R^1$ has the same meaning as in the formula I, is allowed to react with a primary alkylamine of the general formula $NH_2R^2$ in which $R^2$ has the same meaning as in the formula I, in the medium of a polar solvent.

3. A method according to claim 2, characterised in that the reaction takes place in aqueous medium for 24 hours at room temperature.

4. A method of preparing substituted aryloxyaminopropanols of the general formula I according to claim 1, characterised in that an alkyl ester of 3-acetyl-4[(2,3-epoxy)-propoxy]phenylcarbamic acid of the general formula III

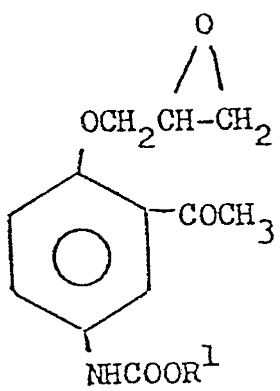

(III)

in which $R^1$ has the same meaning as in the formula I, is allowed to react with a primary alkylamine of the general formula $NH_2R^2$ in which $R^2$ has the same meaning as in the formula I, in the medium of a polar solvent at the boiling temperature of the reaction mixture.

5. A method according to claim 4, characterised in that the polar solvent is an alkanol with 1 to 3 carbon atoms.

6. A method of preparing substituted aryloxyaminopropanols of the general formula I according to claim 1, characterised in that an alkyl ester of 3-acetyl-4-hydroxyphenylcarbamic acid of the general formula IV

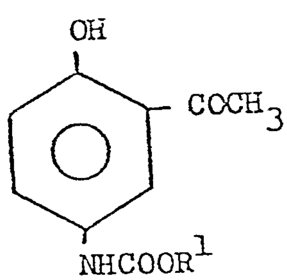

(IV)

in which $R^1$ has the same meaning as in the formula I, is allowed to react with 1-alkylamino-2,3-epoxypropane of the general formula V

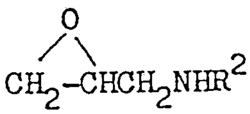

(V)

in which $R^2$ has the same meaning as in the formula I, in the medium of a polar solvent, the reaction being catalyzed by an alkali metal hydroxide.

7. A method according to claim 6, characterised in that the reaction takes place in aqueous medium at room temperature and is catalyzed by sodium hydroxide.

8. A method of preparing substituted aryloxyaminopropanols of the general formula I according to claim 1, characterised in that an alkyl ester of 3-acetyl-4-hydroxyphenylcarbamic acid of the general formula IV in which $R^1$ has the same meaning as in the formula I, is allowed to react with 1-alkylamino-3-chloro-2-propanol of the general formula VI

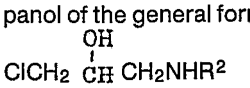

(VI)

in which $R^2$ has the same meaning as in the formula I, in a polar solvent medium, the reaction being catalyzed by an alkali metal hydroxide.

9. A method according to claim 8, characterised in that the reaction takes place in aqueous medium in a sealed tube or an autoclave at a temperature of from 100 to 120°C and that it is catalyzed by sodium hydroxide.